# EUROPÄISCHE PATENTANMELDUNG

(11) **EP 1 992 276 A1**
(43) Veröffentlichungstag der Anmeldung: **19.11.2008**
(21) Anmeldenummer: 08103840.8
(22) Anmeldetag: 06.05.2008
(51) Int. Cl.: A61B 3/113, A61B 3/12, A61B 3/15, A61B 3/00

(54) **Funduskamera**

(30) Priorität: 18.05.2007 DE 102007023270
(71) Anmelder: LINOS Photonics GmbH & Co. KG, 37081 Göttingen (DE)
(72) Erfinder: Kasper, Axel, 81539, München (DE); Zinter, John Robert, Rochester, NY NY 14618 (US)
(74) Vertreter: Taresch, Gudrun

(57) **Zusammenfassung**

Die Erfindung geht aus von einer Funduskamera, zum Betrachten eines Auges (11). Deren Komponenten, eine Beleuchtungseinrichtung (1) zum Beleuchten eines Bildfeldes des Augenhintergrundes (12) und eine Bildaufnahmeeinrichtung (14) auf die der Augenhintergrund (12) über eine Abbildungseinrichtung (10) abgebildet wird, sind konfokal angeordnet. Erfindungsgemäß ist die Beleuchtungseinrichtung (1) so strukturiert, dass in dem von ihr beleuchteten Bildfeld auf dem Augenhintergrund (12) eine periodische Lichtstruktur erzeugbar ist. Ferner ist ein Versatzmittel (21) vorhanden, zum Versetzen der Struktur um weniger als eine Periode. Die Bildaufnahmeeinrichtung (14) ist mit einer Auswerteinrichtung (22) verbunden, um wenigstens drei mit zueinander versetzter Struktur beleuchtete Aufnahmen zu einer Aufnahme zusammen zu fassen. Hierdurch kann aus den drei Aufnahmen mit überlagerter Struktur eine unstrukturierte, extrem scharfe Aufnahme des Augenhintergrundes (12) gewonnen werden.

## Beschreibung

Die Erfindung betrifft eine Funduskamera nach dem Oberbegriff von Anspruch 1.

Für die Beobachtung des Augenhintergrundes sind grundsätzlich zwei unterschiedliche Verfahren bekannt.

Beim herkömmlichen Verfahren wird der Augenhintergrund mit Hilfe einer von einer Lichtquelle ausgehenden Strahlung ausgeleuchtet und an Hand des von dort reflektierten oder emittierten Lichts über eine Zwischenabbildung eine Abbildung auf einen Sensor durchgeführt. Hierbei besteht jedoch die Schwierigkeit, dass die Beleuchtung und die Beobachtung durch die Augenpupille erfolgen muss, an der Reflexe auftreten und die Abbildungsfehler erzeugt.

Diese Art der Funduskameras konnte dadurch maßgeblich verbessert werden, dass die Lichtquelle und der Detektor so angeordnet wurden, dass Anregungs- und Detektorfokus konfokal liegen. Hierdurch kann optische Information, die nicht aus der Fokalebene kommt gut unterdrückt werden, was die Bildqualität der Aufnahmen deutlich erhöht hat. Dennoch ist auch dieses Ergebnis für manche Details nicht ausreichend.

Deshalb wurde ein weiteres Verfahren entwickelt, bei dem der Augenhintergrund nicht großflächig ausgeleuchtet, sondern mit einem fokussierten Lichtstrahl abgetastet und das reflektierte Licht mit einem Sensor erfasst und der Abtastsequenz zugewiesen wird. Die hierfür verwendeten Geräte zur Fundusabbildung werden als Scanning Laser Ophtalmoscope oder Retinal Scanner bezeichnet. Nachteilig an diesem Verfahren ist jedoch, dass der hierfür erforderliche Aufbau relativ aufwändig und teuer ist und dass es auf Grund der Punkt für Punkt Abtastung zu einem Zeitverzug kommt, der insbesondere auf Grund der Augenbewegungen zu verfälschten Ergebnissen führt. Um dieser Problematik entgegen zu wirken wurden verschiedenste Vorschläge gemacht, welche das System an sich jedoch weiter verteuern.
Der Erfindung liegt die Aufgabe zugrunde, eine Funduskamera zu entwickeln, die kostengünstig herstellbar ist und gleichzeitig qualitativ hochwertige Bilder des Augenhintergrundes erzeugt.

Gelöst wird die Aufgabe gemäß der Erfindung durch eine Vorrichtung mit den Merkmalen von Anspruch 1.

Erfindungsgemäß wird eine, den Augenhintergrund flächig abbildende, Funduskamera mit einer strukturierten Beleuchtung ausgestattet. Mittels der strukturierten Beleuchtung wird auf dem Augenhintergrund ein periodisches Lichtintensitätsmuster, also eine sich wiederholende Folge von Hell-Dunkel-Übergängen, erzeugt, welche nur in der Fokalebene der Bildaufnahmeeinrichtung, welche der Fokalebene der Beleuchtungseinrichtung entspricht ein scharfes Bild erzeugt. Strukturen des Augenhintergrunds, welche genau in der Fokalebene der Beleuchtungseinrichtung liegen, weisen in der Aufnahme eine der Beleuchtungsstruktur entsprechende Modulation des Bildsignals auf. Strukturen des Augenhintergrunds, welche in einer anderen Tiefenebene liegen, ergeben auf der Aufnahme eine erheblich geringere Modulation des Bildsignals. Dem vollständig durchmodulierten Bild aus der Fokalebene der Beleuchtungseinrichtung sind in der realen Aufnahme also die nur schwach modulierten Bildsignale überlagert, die nicht aus der Fokalebene stammen.

Um die Aufnahme zu bereinigen und in eine Aufnahme umzuwandeln, in der zum einen die Beleuchtungsstruktur nicht mehr sichtbar ist, und in der zum anderen alle Bildsignale elimiert sind, die nicht aus der Fokalebene stammen, werden unter Beibehaltung des Fokus mindestens zwei weitere Flächenaufnahmen erzeugt, bei denen jedoch die strukturierte Beleuchtung so verändert wird, dass die periodische Lichtstruktur um jeweils weniger als eine Periode gegenüber der vorherigen Aufnahme versetzt ist. In einer vorteilhaften Ausführungsform der Erfindung ist der Versatz dabei jeweils äquidistant und der Verschiebeweg entspricht bei einer Anzahl von n Teilaunahmen genau einem n-tel der Periode der Beleuchtungsstruktur. Werden diese wenigstens drei mit versetzter Struktur erzeugten Aufnahmen miteinander verrechnet, so können alle Störsignale aus der Aufnahme entfernt und letztlich eine vollflächige, extrem scharfe, hoch auflösende Aufnahme einer Ebene des Augenhintergrundes abgeleitet werden, wie sie bislang nur mit Retinal Scannern gewonnen werden konnten. Ein Verfahren zum Verarbeiten der Einzelaufnahmen zu einer korrigierten Gesamtaufnahme ist beispielsweise aus der EP 0 972 220 B1 bekannt. Mit dieser erfindungsgemäßen Erweiterung kann also im wesentlichen unter Beibehaltung des kostengünstig herstellbaren, schnell und effizient arbeitenden Aufbaus einer klassischen Funduskamera, zur Flächenabtastung des Augenhintergrundes, eine Aufnahmequalität erreicht werden, wie sie ansonsten nur mit den weitaus teureren und aufwändigeren Optiken eines Retinal Scanners möglich ist. Hierzu ist lediglich eine Modifikation der Beleuchtungseinheit und eine Aufnahme und Auswertung mehrerer Aufnahmen notwendig.

Ein wesentlicher Aspekt der Erfindung, der sich auf den Einsatz in der Funduskamera bezieht, beschäftigt sich mit der Tatsache, dass das Auge während der Aufnahmen bewegt werden kann. Somit ist, die strukturierte Beleuchtungseinrichtung so auszubilden, dass der Versatz der Struktur in der extrem kurzen Zeitspanne von weniger als einer halben Sekunde stattfinden kann. Hierdurch ist gewährleistet, dass die wenigstens drei Einzelaufnahmen, welche zu einer einzelnen Aufnahme zusammengefasst werden innerhalb einer Zeitspanne von einer Sekunde aufgenommen werden können. Noch vorteilhafter ist es, wenn die Versatzzeit auf weniger als 0,1 Sekunde reduziert werden kann, so dass die Aufnahmezeit der drei zu vereinbarenden Aufnahmen weniger als 0,2 Sekunden beträgt. Hierdurch kann nahezu ausgeschlossen werden, dass eine Bewegung des Auges während der Aufnahmen das Ergebnis verfälscht. Indem die drei zusammen zu fassenden Aufnahmen extrem zeitnah erstellt werden, ist es möglich einen Algorithmus wie den in der EP 0 972 220 B1 vorgeschlagenen in einer Funduskamera zu realisieren.

In einer vorteilhaften Ausführungsform wird die periodische Lichtstruktur auf dem Augenhintergrund erzeugt, indem eine Maske, ein Gitter, oder ein Filter im Beleuchtungsstrahlengang angeordnet ist. Dieses Strukturelement wird vorteilhafter Weise mittels eines Aktors zwischen den Aufnahmen im Strahlengang verschoben. Der Aktor kann beispielsweise als Piezoaktor oder Magnetostriktiver Aktor ausgebildet sein. Derartige Aktoren sind kostengünstig und zuverlässig. Es wäre jedoch auch vorstellbar, dass die Lichtquelle an sich eine periodische Struktur aufweist und die gesamte Lichtquelle mittels eines Aktors bewegbar ist. Ein derartiger Aktor erfüllt im Allgemeinen die Voraussetzungen, die für den notwendigen schnellen Versatz unerlässlich sind.

In einer besonders vorteilhaften Ausführungsform wird die Augenbewegung, welche im vorherigen Ausführungsbeispiel als problematisch erkannt wurde, gezielt eingesetzt wird. Während der Bewegung des Auges bewegt sich die Beleuchtungsstruktur auf dem Augenhintergrund ohnehin. Damit kann auf jede weitere Bewegung der Beleuchtungsstruktur verzichtet werden, wenn die Bewegung des Augenhintergrundes entweder konkret gesteuert oder zumindest nachvollzogen werden kann oder ein Algorithmus zum Auswerten der Bilder auch mit willkürlichen Bewegungen arbeiten kann. Dieser einzigartige Vorteil ergibt sich erst durch den Einsatz einer strukturierten Beleuchtung in einer Funduskamera und wurde hier erstmals erkannt. Insbesondere ist die Augenbewegung sehr schnell, so dass bei dieser Variante eine besonders schnelle Abfolge von mehreren Aufnahmen möglich ist und so extrem zeitnahe Auswertungen möglich werden.

So kann die Augenbewegung über eine konkrete Bewegung eines einer Funduskamera oftmals eigenen Fixiertargets so gesteuert werden, dass die Beleuchtungsstruktur bei der Bewegung, dem Fixiertarget folgend, auf dem Augenhintergrund um konkrete, festgelegte Wege verschoben wird. Diese sind dann ebenso bekannt, wie bei einer Verschiebung der Beleuchtungsstruktur selbst und können bei der Auswertung einfach berücksichtigt werden. Hierfür wird der Auswertung übermittelt, inwieweit das Fixiertarget verschoben wurde.

In einer weiteren vorteilhaften Ausführungsform wird eine willkürliche Augenbewegung zugelassen, diese jedoch aufgezeichnet oder aus den aufgenommenen Bilddaten ermittelt. Zur Ermittlung des Verschiebungsvektors können beispielsweise Kantendetektionsverfahren oder andere bekannte Algorithmen der Bildverarbeitung verwendet werden. Bei dieser Ausführungsform ist als Modifikation der Funduskamera also lediglich eine statische strukturierte Beleuchtung notwenig und eine Auswerteinheit, die eine Folge von aufgenommenen Bildern unter Berücksichtigung der Verschiebung verarbeiten kann.

In einer vorteilhaften Ausführungsform ist die Bildaufnahmeeinrichtung durch eine Auflösung von wenigstens einem Megapixel gekennzeichnet. Nur durch eine derartig hohe Auflösung kann gewährleistet werden, dass eine für eine Diagnose ausreichende Detailauflösung der Aufnahme erreicht werden kann und gleichzeitig ein für einen Überblick ausreichend großer Bildbereich des Augenhintergrundes mit einer einzigen Aufnahme erfasst werden kann. Nur eine derartig hohe Auflösung gewährleistet, dass die durch den Einsatz einer strukturierten Beleuchtung zu erreichende Detailgenauigkeit und Bildschärfe realisierbar ist.

In einer weiteren vorteilhaften Ausführungsform der Erfindung sind Elemente bzw. Anordnungen von Elementen vorgesehen, die Reflexe des Beleuchtungslichts, welche als Störstrahlung die Qualität der Aufnahme maßgeblich beeinträchtigen könnten, unterdrücken. Generell wird bei dem in der EP 0 972 220 B1 offenbarten Verfahren davon ausgegangen, dass Bildinformation, welche nicht aus der Schärfeebene kommt, ohnehin unterdrückt wird und keinen Einfluss auf die Aufnahmequalität hat. Wird diese Form der strukturierten Beleuchtung jedoch in einer Funduskamera eingesetzt, so kann diese Annahme, wie sich überraschenderweise herausgestellt hat, nicht gehalten werden. In einer Funduskamera besteht nämlich die Problematik, dass das vom Augenhintergrund reflektierte Nutzsignal, welches das eigentliche aufzunehmende Bild darstellt äußerst schwach gegenüber dem von Reflexen z.B. innerhalb des Auges verursachten Gesamtsignals ist. Deshalb wurde erfindungsgemäß vorgeschlagen, eine weitere Maßnahme zu ergreifen, um nur das geringe Signal des an der zu beobachtenden Netzhaut reflektierten Lichts herauszufiltern. Hierdurch konnte die Bildqualität nochmals maßgeblich verbessert werden.

Vorteilhafterweise sind zwischen Lichtquelle und aufzunehmendem Objekt sowie zwischen diesem und der Bildaufnahmeeinrichtung jeweils Polarisatoren angeordnet, wobei die Polarisatoren orthogonal zueinander stehen, sogenannte gekreuzte Polarisatoren. Vorzugsweise handelt es sich hierbei um Polarisatoren mit sehr hohem Polarisationsgrad. Dabei wird die Tatsache ausgenützt, dass die Reflektion bzw. Rückstreuung an der Netzhaut depolarisierend auf das aufzunehmende Licht wirkt, während dies auf viele andere störende Reflexe (z.B. von Linsenflächen des optischen Systems oder von der Cornea) nicht zutrifft.

Um den Transmissionswirkungsgrad für das Nutzlicht zu erhöhen ist es vorteilhaft, einen Strahlteiler, welcher üblicherweise in konfokalen Fokuskameras verwendet wird, um Beleuchtungs- und Aufnahmestrahlengang zu trennen, polarisierend auszuführen.

Nachteilig an der Verwendung von Polarisatoren ist jedoch, dass diese einen großen Anteil des durch sie hindurch tretenden Lichtes absorbieren. Deshalb ist in einer weiteren vorteilhaften Ausführungsform vorgesehen, in dem von Beleuchtung und Beobachtung gemeinsam genutzten Strahlengang eine, mehrere oder alle Linsen dezentriert und/oder verkippt anzuordnen. Dadurch können unerwünschte Reflexe von der Beleuchtung nicht zum Aufnahmesensor gelangen, sondern werden an geeigneten Stellen in der optischen Anordnung absorbiert.

Mit der erfindungsgemäßen Anordnung ist es möglich und vorteilhaft, einen hochauflösenden Tiefenscan des Augenhintergrundes durchzuführen, indem ein optisches Element im Beleuchtungs- und Aufnahmestrahlengang angeordnet ist, welches so aufgebaut ist, dass mit Hilfe dieses Elements die Fokusebene auf dem aufzunehmenden Augenhintergrund verschoben werden kann. Dies kann durch eine bewegliche Linse realisiert sein, aber auch beispielsweise durch ein optisch aktives Element, dessen Brechkraft veränderbar ist. Indem der Fokus räumlich verschoben wird, wird gewissermaßen der Augenhintergrund in seiner Tiefe abgetastet, wobei jeweils an jeder neuen Fokusposition wenigstens drei Aufnahmen zu erstellen sind, welche dann zu je einer Gesamtaufnahme zusammengefasst werden.

In einer weiteren bevorzugten Ausführungsform ist die Auswerteinrichtung so ausgestattet, dass die in unterschiedlichen Fokusebenen erzeugten Aufnahmen auf solche charakteristische Merkmale hin untersucht werden, welche auf allen diesen Aufnahmen wieder zu finden sind. So kann eine räumliche Zuordnung der Bilder vorgenommen werden und dadurch können Verschiebungen ausgeglichen werden, welche durch Augenbewegungen verursacht werden. Während es durch ein schnelles Aneinanderreihen der jeweils drei Einzelaufnahmen erfindungsgemäß gewährleistet ist, derartige Verschiebungen durch Augenbewegungen zu vermeiden, müssen diese bei einer Aneinanderfügung der in unterschiedlichen Fokusebenen erzeugten Aufnahmen berücksichtigt und anderweitig bereinigt werden, da die Fokusverschiebung und erneute Aufnahme jeweils dreier Bilder soviel Zeit in Anspruch nimmt, dass Verschiebungen aufgrund von Augenbewegungen unvermeidlich sind. Erfindungsgemäß wird hier mit Mitteln der Bildverarbeitung gearbeitet.

In einer erfindungsgemäßen Funduskamera sind vorteilhafter Weise in der Ausgangsposition vor dem Tiefenscan die abzubildende Beleuchtungsstruktur, die Bildaufnahmeeinrichtung und ein Fixiertarget einer Akkommodationseinrichtung zueinander konjugiert, d. h., alle sind gleichzeitig auf die Netzhaut fokussiert. Hierdurch kann gewährleistet werden, dass das Auge so akkommodiert ist, dass der beleuchtete Bereich der Netzhaut scharf auf die Aufnahmeeinrichtung abgebildet werden kann. Der Aufbau zur Ausführung eines Tiefenscans wird nun erfindungsgemäß so realisiert, dass die verschiebbare Linse so im Strahlengang angeordnet ist, dass sie nur die Fokusposition von Struktur und Aufnahmeeinrichtung verändert, während der Strahlengang und damit der Fokus der Akkommodationseinrichtung unverändert bleibt. Hierdurch wird verhindert, dass die Akkommodation des Auges gegen den Tiefenscan arbeitet. (Während des Tiefenscans ist die Schärfenebene des Targets nicht mehr mit den Schärfebenen der Beleuchtung und der Abbildung konjugiert.)

In einer weiteren vorteilhaften Ausführungsform der Erfindung ist das Fixiertarget als flächiges Objekt ausgebildet, während in herkömmlichen Funduskameras nur ein punktförmiges Objekt zentral auf der optischen Achse angeordnet ist. Dadurch wird ermöglicht, bei der Aufnahme auch seitliche Positionen im Augeninneren anzusteuern, um neben dem zentralen Bereich der Netzhaut auch periphere Bereiche zu beobachten. Hierfür kann das Fixiertarget beispielsweise als frei ansteuerbares LED- oder OLED-Display ausgebildet sein.

In einer weiteren vorteilhaften Ausführungsform der Erfindung ist die Funduskamera so ausgeführt, dass Aufnahmen in verschiedenen Farben erstellt werden können, was für diverse Diagnosezwecke gewünscht ist. Dies kann dadurch ermöglicht werden, dass die Beleuchtungseinrichtung Licht in unterschiedlichen Farben emittieren kann, wie dies bspw. bei einem Array verschiedenfarbiger LEDs möglich ist. Diese haben den Vorteil, dass sie extrem schnell schaltbar sind, so dass es möglich ist, jeweils drei Aufnahmen in unterschiedlichen Farben zu machen, welche dann miteinander verglichen werden können, ohne dass es zu Beeinträchtigungen aufgrund von Augenbewegungen zwischen den Aufnahmen kommt. Es wäre jedoch auch möglich, eine weiße Lichtquelle zu verwenden und für Aufnahmen in unterschiedlichen Farben Farbfilter in den Beleuchtungsstrahlengang einzubringen. Eine weitere Möglichkeit ist es, für die Bildaufnahmeeinrichtung einen Farbsensor zu verwenden um so Aufnahmen in gezielt unterschiedlichen Farben tätigen zu können. Hierdurch ist es auch möglich, Fluoreszenzaufnahmen mittels der erfindungsgemäßen Funduskamera zu realisieren. Wichtig ist nur, dass immer jeweils mindestens drei Aufnahmen in einer Farbe gemacht und zusammengefasst werden.

In einer weiteren vorteilhaften Ausführungsform der Erfindung ist eine Iriskamera als Bestandteil der Funduskamera vorgesehen, welche die Pupille und die Iris des Patienten beobachtet. Mit Hilfe dieser Iriskamera kann die gesamte Funduskamera in drei Dimensionen zum Auge positioniert werden. Die Justage dieser Kamera zum Auge in den drei Raumrichtungen erfolgt transversal über die Zentrierung beispielsweise eines Fadenkreuzes zur Pupille und in Abstandsrichtung über die scharfe Abbildung, beispielsweise der Iris oder des Pupillenrandes.

Weitere Einzelheiten und Vorteile der Erfindung ergeben sich aus den Unteransprüchen im Zusammenhang mit der Beschreibung eines Ausführungsbeispiels, das anhand der Zeichnungen eingehend erläutert wird.

Es zeigen:
- Fig. 1: schematisch den Aufbau einer Funduskamera mit strukturierter Beleuchtung
- Fig. 2: ein weiteres Ausführungsbeispiel einer Funduskamera mit strukturierter Beleuchtung.

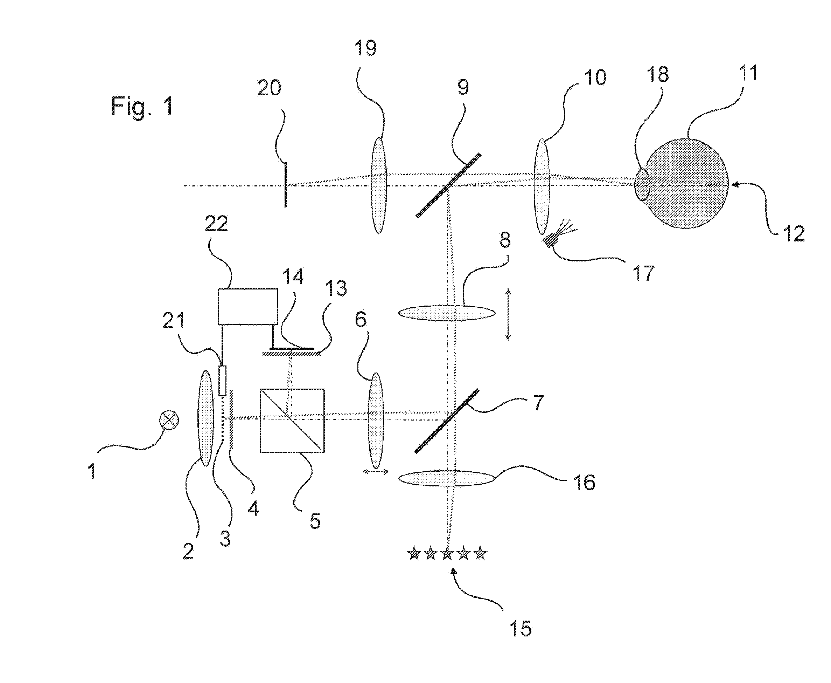

Die, in Fig. 1 dargestellte, erfindungsgemäße, für hoch auflösende Flächenaufnahmen der Netzhaut geeignete Funduskamera weist zur Beleuchtung der Netzhaut eine Lichtquelle 1 auf, deren Licht über eine Kondensorlinse 2 und eine Maske 3 durch einen Polarisator (z.B. eine Polarisationsfolie) 4, über einen polarisierenden Strahlteiler 5 und eine Abbildungslinse 6 (das ist die bewegliche Linse für den Tiefenscan, die nur Beleuchtung und Abbildung betrifft), einen dichroitischen Akkommodationsstrahlteiler 7 zum Abspalten des Akkommodationsstrahlengangs, eine Diopter-Linse 8 zur Anpassung der Kamera an die individuelle Fehlsichtigkeit des Patienten, einen dichroitischen Strahlteiler 9 für den Strahlengang der Iriskamera und die Objektivlinse 10 durch die Pupille des Auges 11 auf die Netzhaut 12 abgebildet wird. Genau genommen wird die von der Lichtquelle 1 über die Kondensorlinse 2 beleuchtete Maske 3, bzw. das durch sie erzeugte Hell-DunkelMuster auf die Netzhaut 12 abgebildet. Das von der Netzhaut 12 reflektierte bzw. zurückgestreute Licht wird über den mit dem Beleuchtungsstrahlengang identischen Abbildungsstrahlengang bis zum polarisierenden Strahlteiler 5 zurückgeführt und von diesem über einen aufnahmeseitigen Polarisator (z.B. eine Polarisationsfolie) 13 auf einen Aufnahmesensor 14 gelenkt. Über den Akkommodationsstrahlteiler 7 wird das von einem Fixiertarget 15 emittierte und über eine weitere Abbildungslinse 16 fokussierte Licht in den Strahlengang eingekoppelt und ebenfalls auf die Netzhaut 12 geführt, so dass das Fixiertarget auf die Netzhaut abgebildet wird. Ferner ist eine LED-Lichtquelle 17 zur diffusen Beleuchtung der Iris 18 vorgesehen, wobei das von der Iris 18 reflektierte Licht über den Strahlteiler 9 der Iriskamera aus dem Strahlengang ausgekoppelt und über eine Iriskamera-Linse 19 auf einen Iriskamera-Sensor 20 abgebildet wird.

Als Lichtquelle 1 eignet sich beispielsweise eine Halogenlampe, deren Licht für Farbaufnahmen über nicht dargestellte Farbfilter oder über einen als Farbsensor ausgebildeten Sensors 14 zur Realisierung von Farbaufnahmen aufgespaltet werden kann. Ebenso als Lichtquelle 1 geeignet sind LEDs. Diese können bezüglich Ihres Spektrums so gewählt werden, dass farbselektive Aufnahmen oder im Falle von weißen LEDs mit einem Farbsensor Farbaufnahmen möglich sind. Verwendet man als Sensor 14 einen Farbsensor, so ist zu beachten, dass die Auflösung des Sensors 14 unter Umständen proportional zur Anzahl der Farben erhöht werden muss, um zu gewährleisten, dass in jeder Farbaufnahme eine Auflösung von wenigstens einem Megapixel zur Verfügung steht.

Das Licht der Lichtquelle 1 wird über einen Kondensor 2 gleichmäßig auf eine Maske 3 verteilt. Die Maske 3 ist in der hier beschriebenen bevorzugten Ausführungsform als periodisches Liniengitter realisiert und steht mit einem Piezoaktor 21 in Verbindung, über den das Liniengitter um jeweils weniger als einen Gitterabstand versetzt werden kann. Aufgrund dieser Bewegung verschiebt sich das über den Abbildungsstrahlengang auf der Netzhaut 12 abgebildete Streifenmuster jeweils um weniger als eine Periode. Hierdurch ist es möglich, mehrere Aufnahmen eines Bereichs der Netzhaut 12 nacheinander zu machen, denen jeweils dasselbe Streifenmuster, aber jeweils um weniger als eine Periode verschoben zueinander überlagert. Die am Sensor 14 aufgenommenen Bilder werden zu einem Computer 22 weitergeleitet, an dem sie zu einer einzigen streifenfreien Gesamtaufnahme der Netzhaut verarbeitet werden. Über den Computer 22 wird eine Verbindung zum Aktor 21 hergestellt, so dass die Aufnahmen am Sensor 14 mit der Verschiebung des Piezoaktors 21 korreliert werden können. Das durch die Maske 3 hindurch tretende Beleuchtungslicht wird an einem Polarisator 4 polarisiert, so dass nur polarisiertes Licht die Netzhaut 12 erreicht. Dieses polarisierte Licht kann nicht direkt auf den Sensor 14 gelangen, da vor diesem ein weiterer Polarisator 13 angeordnet ist, dessen Polarisationsrichtung senkrecht zu der des ersten Polarisators 4 ist. Hierdurch wird verhindert, dass Licht welches an einem der vielen optischen Elemente reflektiert wird oder durch einen Reflex des Auges, welches für die Aufnahme extrem gut ausgeleuchtet werden muss, auf den Sensor 14 fällt und die Aufnahme der Netzhaut 12 verfälscht. Das aufzunehmende Licht der Netzhaut 12 selbst wird bei der Reflektion bzw. Rückstreuung an der Netzhaut 12 weitgehend depolarisiert, so dass es zumindest teilweise durch den Polarisator 13 hindurch auf den Sensor 14 treten kann. Durch die Polarisation von Beleuchtungs- und Abbildungslicht wird also gewissermaßen aus dem hohen Anteil an Störlicht, das über Reflexe an irgendwelchen Komponenten in den Abbildungsstrahlengang gelangt der sehr geringe Anteil an Nutzlicht, das direkt an der Netzhaut 12 reflektiert bzw. zurückgestreut wird herausgefiltert. Dem liegt die Annahme zugrunde, dass der, an der Netzhaut 12 reflektierte bzw. zurückgestreute Anteil der Strahlung bei der Reflektion depolarisiert wird.

Die Abbildungslinse 6 ist entlang des Strahlengangs verschiebbar, so dass der Fokus von Beleuchtungs- und Abbildungsstrahlengang gleichermaßen verändert werden kann. Hierdurch ist es möglich, entlang der Tiefe der Netzhaut 12 verschiedene Fokalebenen abzutasten und somit jeweils drei Flächenaufnahmen in unterschiedlicher Tiefe zu erzeugen, so dass insgesamt ein Tiefenscan der Netzhaut 12 erstellt werden kann. Die Abbildungslinse 6 ist vor dem Strahlteiler 7 angeordnet, über den der Strahlengang des Fixiertargets 15, der zur Akkommodation des Auges dient, im Beleuchtungs- und Abbildungsstrahlengang eingeblendet wird. Hierdurch ist gewährleistet, dass die Verschiebung der Linse 6 auf den Akkommodationsstrahlengang keinen Einfluss hat, so dass die Akkommodation des Auges nicht geändert wird, während durch die Verschiebung der Linse 6 die Abtastung der Tiefe der Netzhaut 12 erfolgt. Über eine weitere verschiebbare Linse 8 erfolgt die Dioptrien-Einstellung zur Anpassung der Abbildung der Kamera an die individuelle Fehlsichtigkeit des Patientenauges, so dass jeweils eine fehlerfreie Scharfstellung auf die Netzhaut 12 gewährleistet ist. Hierzu ist es notwendig, dass das Auge gut auf das Fixiertarget 15, welches beispielsweise als Flächen-LED ausgebildet ist, akkommodiert ist.

Die zusätzliche LED-Lichtquelle 17 dient der diffusen Beleuchtung der Iris 18 um für die Positionierung der Funduskamera zum Patientenauge ein helles und gut erkennbares Bild der Iris zur Verfügung zu stellen. Das von der Iris 18 reflektierte bzw. zurückgestreute Licht wird über den Strahlteiler 9 der Iriskamera aus dem Strahlengang ausgekoppelt und über eine Iriskamera-Linse 19 auf einen Iriskamera-Sensor 20 abgebildet. Die Schärfeebene der Iris-Kamera muss dabei so eingestellt sein, dass bei einer scharfen Abbildung der Iris oder besonders des Pupillenrandes der gewünschte Arbeitsabstand der Funduskamera zum Auge des Patienten erreicht wird. Über die Zentrierung des Pupillenbildes im Bildfeld der Iriskamera eventuell auch unter Zuhilfenahme eines Fadenkreuzes wird die Funduskamera so positioniert, dass der Abbildungsstrahlengang für die Fundusabbildung optimal zum Patientenauge ausgerichtet ist. Die Wellenlänge der Iris-Beleuchtung wird bevorzugt so gewählt, dass sie zum einen in einem anderen Bereich des Spektrums liegt als das Beleuchtungsspektrum für die Funduskamera und die Beleuchtung des Fixiertargets und zum anderen so am Rand des sichtbaren Spektralbereiches liegt, dass wegen der verminderten Augenempfindlichkeit die Blendwirkung minimal ist. Ein bevorzugter Spektralbereich für die Iris-Beleuchtung liegt im nahen Infrarot.

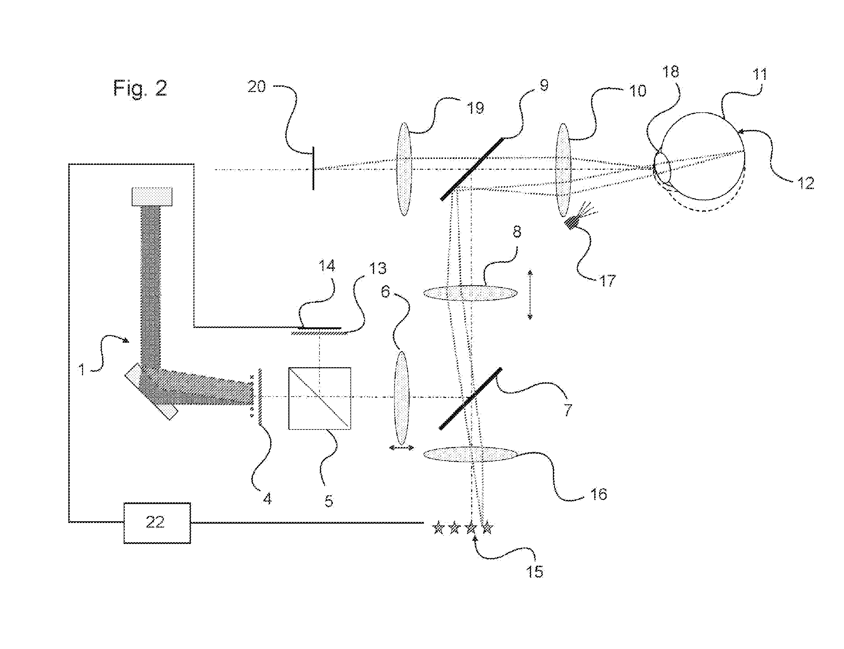

Auch die in der Fig. 2 dargestellte, erfindungsgemäße, für hoch auflösende Flächenaufnahmen der Netzhaut geeignete Funduskamera weist zur Beleuchtung der Netzhaut eine Lichtquelle 1 auf. Diese ist hier jedoch als Interferrometer ausgebildet, so dass durch die Interferenz kohärenter Strahlen gleich ein Linienmuster erzeugt wird, welches dann auf den Augenhintergrund abgebildet werden kann. Die Abbildung erfolgt über einen polarisierenden Strahlteiler 5 und eine Abbildungslinse 6, einen dichroitischen Akkommodationsstrahlteiler 7 zum Abspalten des Akkommodationsstrahlengangs, eine Diopter-Linse 8 zur Anpassung der Kamera an die individuelle Fehlsichtigkeit des Patienten, einen dichroitischen Strahlteiler 9 für den Strahlengang der Iriskamera und die Objektivlinse 10 durch die Pupille des Auges 11 auf die Netzhaut 12. Das von der Netzhaut 12 reflektierte bzw. zurückgestreute Licht wird über den mit dem Beleuchtungsstrahlengang identischen Abbildungsstrahlengang bis zum polarisierenden Strahlteiler 5 zurückgeführt und von diesem über einen aufnahmeseitigen Polarisator (z.B. eine Polarisationsfolie) 13 auf einen Aufnahmesensor 14 gelenkt. Über den Akkommodationsstrahlteiler 7 wird das von einem Fixiertarget 15 emittierte und über eine weitere Abbildungslinse 16 fokussierte Licht in den Strahlengang eingekoppelt und ebenfalls auf die Netzhaut 12 geführt, so dass das Fixiertarget auf die Netzhaut abgebildet wird. Ferner ist eine LED-Lichtquelle 17 zur diffusen Beleuchtung der Iris 18 vorgesehen, wobei das von der Iris 18 reflektierte Licht über den Strahlteiler 9 der Iriskamera aus dem Strahlengang ausgekoppelt und über eine Iriskamera-Linse 19 auf einen Iriskamera-Sensor 20 abgebildet wird.

Die von der Lichtquelle 1 erzeugte Linienstruktur bleibt in diesem Ausführungsbeispiel immer an einem Ort. Das Versetzen der Struktur erfolgt durch die Bewegung des Auges 11. Aufgrund dieser Bewegung verschiebt sich das über den Abbildungsstrahlengang auf der Netzhaut 12 abgebildete Streifenmuster jeweils um weniger als eine Periode. Hierdurch ist es möglich, mehrere Aufnahmen eines Bereichs der Netzhaut 12 nacheinander zu machen, denen jeweils dasselbe Streifenmuster, aber jeweils um weniger als eine Periode verschoben zueinander überlagert. Die am Sensor 14 aufgenommenen Bilder werden zu einem Computer 22 weitergeleitet, an dem sie zu einer einzigen streifenfreien Gesamtaufnahme der Netzhaut 12 verarbeitet werden. Über den Computer 22 wird eine Verbindung zum Fixiertarget 15 hergestellt, so dass die Aufnahmen am Sensor 14 mit der Verschiebung des Fixiertargets korreliert werden können. Sobald das Fixiertarget 15 verschoben wird, bewegt sich das Auge 11, um dieser Verschiebung zu folgen. Dadurch wird erreicht, dass das Streifenmuster auf der Netzhaut 12 wandert. Über eine gezielte Bewegung des Fixiertargets 15 kann somit ein gezieltes Versetzen des Streifenmusters bewirkt werden. In diesem Ausführungsbeispiel erfolgt also der Versatz des Streifenmusters auf der Netzhaut nicht über einen Versatz des Musters in der Beleuchtungseinheit sondern über einen Versatz der Netzhaut 12, welcher hier durch den Versatz der Fixiertargets 15 ausgelöst wird.

Alle anderen Komponenten, wie z.B. Polarisatoren 4 und 13 und die verschiedensten Linsen erfüllen in diesem Ausführungsbeispiel dieselben Funktionen wie in dem in Fig. 1 gezeigten.

### Bezugszeichenliste:

- 1: Lichtquelle
- 2: Kondensorlinse
- 3: Maske
- 4: Polarisator
- 5: Strahlteiler
- 6: Abbildungslinse für Tiefenscan
- 7: Dichroitischer Strahlteiler für Akkommodationsstrahlengang
- 8: Diopter-Linse
- 9: Dichroitischer Strahlteiler für Iriskamera
- 10: Objektivlinse
- 11: Auge
- 12: Netzhaut
- 13: Polarisator im Abbildungsstrahlengang
- 14: CCD-Sensor
- 15: Fixiertarget
- 16: Abbildungslinse des Akkommodationsstrahlengangs
- 17: LED-Lichtquelle
- 18: Iris
- 19: Iriskamera-Linse
- 20: Iriskamera-Sensor
- 21: Piezoaktor
- 22: Computer

## Patentansprüche

1. Funduskamera, zum Betrachten eines Auges (11), mit einer Beleuchtungseinrichtung (1) zum Beleuchten eines Bildfeldes des Augenhintergrundes (12), welches mittels einer Abbildungseinrichtung 10 auf eine Bildaufnahmeeinrichtung (14) abbildbar ist, wobei Beleuchtungs- und Bildaufnahmefokus konfokal angeordnet sind, **dadurch gekennzeichnet, dass** die Beleuchtungseinrichtung (1) so strukturiert ist, dass in dem von ihr beleuchteten Bildfeld auf dem Augenhintergrund (12) eine periodische Lichtstruktur erzeugbar ist und ein Versatzmittel (21) vorhanden ist, zum Versetzen der Struktur um weniger als eine Periode und die Bildaufnahmeeinrichtung (14) mit einer Auswerteinrichtung (22) verbunden ist, um wenigstens drei mit zueinander versetzter Struktur beleuchtete Aufnahmen zu einer Aufnahme zusammen zu fassen.

2. Funduskamera nach Anspruch 1, **dadurch gekennzeichnet, dass** das Versatzmittel (21) eine Versatzzeit von weniger als 0.5 s realisiert.

3. Funduskamera nach Anspruch 2, **dadurch gekennzeichnet, dass** das Versatzmittel (21) eine Versatzzeit von weniger als 0.1 s realisiert.

4. Funduskamera nach Anspruch 1, 2 oder 3, **dadurch gekennzeichnet, dass** das Versatzmittel (21) der Augenhintergrund (12) ist.

5. Funduskamera nach Anspruch 4, **dadurch gekennzeichnet, dass** die Funduskamera ein über eine Bewegungseinrichtung bewegbares Fixiertarget (15) aufweist, mittels dessen die Bewegung des Augenhintergrundes (12) steuerbar ist.

6. Funduskamera nach Anspruch 5, **dadurch gekennzeichnet, dass** die Bewegungseinrichtung des Fixiertargets (15) mit der Auswerteinrichtung (22) verbunden ist.

7. Funduskamera nach Anspruch 2 oder 3, **dadurch gekennzeichnet, dass** das Versatzmittel (21) ein Aktor ist.

8. Funduskamera nach Anspruch 1, **dadurch gekennzeichnet, dass** eine verschiebbare Maske (3) zur Erzeugung einer bzgl. der Lichtintensität sinusförmig modulierten Struktur auf dem Augenhintergrund (12) vorgesehen ist, wobei die Maske (3) eine Periodizität in nur einer Richtung aufweist.

9. Funduskamera nach Anspruch 1, **dadurch gekennzeichnet, dass** die Bildaufnahmeeinrichtung (14) eine Auflösung von wenigstens 1 MegaPixel aufweist.

10. Funduskamera nach Anspruch 1, **dadurch gekennzeichnet, dass** optische Elemente (4, 6, 8, 10, 13) so angeordnet oder zusätzlich in dem Strahlengang vorgesehen sind dass störende Reflexe durch sie unterdrückt werden.

11. Funduskamera nach Anspruch 10, **dadurch gekennzeichnet, dass** ein Polarisator (4) im Beleuchtungsstrahlengang und ein Polarisator (13) im Aufnahmestrahlengang angeordnet ist.

12. Funduskamera nach Anspruch 11, **dadurch gekennzeichnet, dass** ein polarisierender Strahlteiler (5) im Beleuchtungs- und Aufnahmestrahlengang angeordnet ist.

13. Funduskamera nach Anspruch 10, **dadurch gekennzeichnet, dass** abbildende optische Elemente (6, 8, 10) im Strahlengang dezentriert angeordnet sind.

14. Funduskamera nach Anspruch 10, **dadurch gekennzeichnet, dass** abbildende optische Elemente (6, 8, 10) im Strahlengang verkippt angeordnet sind.

15. Funduskamera nach Anspruch 1, **dadurch gekennzeichnet, dass** eine verschiebbare Linse (6) im Beleuchtungs- und Aufnahmestrahlengang angeordnet ist, um eine räumliche Aufnahme des Augenhintergrundes (12) zu ermöglichen.

16. Funduskamera nach Anspruch 15, **dadurch gekennzeichnet, dass** die Auswerteinrichtung (22) eine Nachführeinrichtung umfasst, in der die jeweils mit unterschiedlichem Linsenfokus erzeugten Aufnahmen analysiert und gegebenenfalls gegeneinander verschoben werden, so dass eine Verschiebung der Bildinformation aufgrund von Augenbewegungen korrigierbar ist.

17. Funduskamera nach Anspruch 16, **dadurch gekennzeichnet, dass** eine Akkommodationseinrichtung vorgesehen und so angeordnet ist, dass die verschiebbare Linse (8) sich außerhalb des Akkommodationsstrahlenganges befindet.

18. Funduskamera nach Anspruch 17, **dadurch gekennzeichnet, dass** das Fixiertarget (15) der Akkommodationseinrichtung räumlich ausgedehnt ist.

19. Funduskamera nach Anspruch 1, **dadurch gekennzeichnet, dass** die Beleuchtungseinrichtung 1 selektiv Licht unterschiedlicher Wellenlängen zum Erstellen farbselektiver Aufnahmen aussenden kann.

20. Funduskamera nach Anspruch 19, **dadurch gekennzeichnet, dass** Farbfilter in den Beleuchtungsstrahlengang einbringbar sind.

21. Funduskamera nach Anspruch 1, **dadurch gekennzeichnet, dass** die Bildaufnahmeeinrichtung (14) einen Farbsensor aufweist.

22. Funduskamera nach Anspruch 1, **dadurch gekennzeichnet, dass** eine Iris-Kamera (17, 19, 20) zum Ausrichten der Funduskamera vorgesehen ist.
